# EUROPEAN PATENT SPECIFICATION

(11) **EP 2 654 617 B1**
(45) Date of publication and mention of the grant of the patent: **03.06.2020**
(21) Application number: 11819115.4
(22) Date of filing: 22.12.2011
(51) Int. Cl.: A61F 2/01

(54) **DEVICE FOR DEFLECTING EMBOLI IN AN AORTA**
VORRICHTUNG ZUR ABLENKUNG VON EMBOLIEN IN EINER AORTA
DISPOSITIF DE DÉVIATION D'EMBOLES DANS UNE AORTE

(30) Priority: 23.12.2010 US 201061426578 P
(43) Date of publication of application: 30.10.2013
(73) Proprietor: Keystone Heart Ltd., Caesarea Business Park 3088900 (IL)
(72) Inventor: SHEZIFI, Yuval, 34752 Haifa (IL); SHIMON, Dov V., 46600 Herzlyia-Pituach (IL); SALMAN, Iddo, 39012 Tirat Ha'Carmel (IL); MIKOVSKY, Tzeela, 52295 Ramat Gan (IL)
(74) Representative: KIPA AB
(86) International application number: PCT/IL2011/000963
(87) International publication number: WO 2012/085916

(56) References cited:
- WO-A1-2010/026240
- WO-A2-2007/129323
- US-A1- 2006 241 678

## Description

### FIELD OF THE INVENTION

Embodiments of the invention relate to deflecting emboli in an aorta to prevent emboli from entering arteries, for example, arteries that lead to the brain.

### BACKGROUND OF THE INVENTION

Devices such as vascular filters or other devices may be inserted into a blood vessel prior to or during a procedure or at another time. Such devices may be inserted by way of a catheter that may be, for example, threaded through a vein or artery, and into, for example, an aorta or other vessel where the device may be released from the catheter and, for example, deployed. The device may filter, deflect, or block emboli or other objects from entering into a blood supply that feeds the brain. A filter according to the introductory portion of claim 1 is disclosed in WO 2007/129323 A2.

### SUMMARY OF THE INVENTION

In one aspect, the invention according to claim 1 features a device for deflecting emboli including a lateral structure to support an emboli filter, the lateral structure having a length, having the filter attached to and extending the length of the lateral structure; a lower member extending downward from the lateral structure in a direction of an ascending aorta, wherein upon installation of the device, the lower member exerts lift on a middle area of the lateral structure; and an upper member extending upwards from the lateral structure, wherein a support portion of the upper member being proximate to the lateral structure is angled towards the ascending aorta, and an anchor portion of the upper member being distal to the lateral structure is angled towards the descending aorta, wherein upon the installation, the upper member limits the lift.

In this aspect, the lateral structure includes a first end and a second end. The first end can include, e.g., a hook configured from a wire of the lateral structure, the hook having a latch to hold a lasso brought into contact with the hook. Also in this aspect, the lower member can include a right segment having a first end attached to the lateral structure, and a left segment having a first end attached to the lateral structure, where a second end of the right segment is unconnected to a second end of the left segment. In certain embodiments, the second end of the right segment can be angled towards the second end of the left segment. Also, the anchor portion of the upper member can taper distal to said lateral structure.

In any of the devices of the invention, the upper member and the lower member can be extensions of a wire entwined around or framing the lateral structure.

Also, any of the lateral structures, upper members, or lower members of the devices of the invention, can be fabricated in whole or part from Nitinol wire or Drawn Filled Tubing (e.g., Drawn Filled Tubing that includes an outer layer of Nitinol and/or a core of tantalum and/or platinum). Furthermore, the filters of the invention can include a mesh (e.g., a mesh fabricated with Nitinol wire, Drawn Filled Tubing (e.g., as described above), or a combination of both) or perforated film. In embodiments where a mesh is present, the filter mesh can be rectilinear (e.g., square) or rhomboid. In embodiments where the pores are rectilinear or rhomboid, one or both lateral dimensions of the pore can be between 50 and 1000 microns (e.g., 100, 200, 300, 400, 500, 600, or more microns). In embodiments where a perforated film is present, the pores formed in the perforated film include a varied or unvaried shape, have a varied or constant density across the film, and/or have a constant or varied size.

Any of the devices of the invention can further include a radiopacity marker (e.g., a bead or clamp).

In some aspects, the support portion of the device includes two support members that slope from the lateral structure to the anchor portion. The slope of each of the two members can be uniform or non-uniform and can include an inflection point, thereby forming a segment of each of the two members proximal to the lateral structure and a segment of the two members distal to the lateral structure. In some embodiments, the segments of each of the two members of the support portion form a medial angle of more than 180° at the inflection point.

The width of the upper member of the device can vary according to the particular configuration. For example, in some embodiments, the distance between the inflection points of the two members is less than the width of the widest region of the lateral structure. In certain embodiments, at the inflection point, the distance between the support members can have, e.g., a width of 100%, 95%, 90%, 85%, 80%, 75%, 70%, 60%, 50%, 40%, 30%, 20%, 10%, or less of the width of the widest region of the lateral structure of the intra-vascular device (e.g., the width can be between 100% and 10%, 80% and 20%, 60% and 20%, 50% and 20%, and 50% and 30% of the widest region of the lateral structure of the intra-vascular device). The anchor portion can have a width, e.g., of 100%, 95%, 90%, 85%, 80%, 75%, 70%, 60%, 50%, 40%, 30%, 20%, 10%, or less of the width of the widest region of the lateral structure of the intra-vascular device (e.g., the width can be between 100% and 10%, 80% and 20%, 60% and 20%, 50% and 20%, and 50% and 30% of the widest region of the lateral structure of the intra-vascular device). Furthermore, the anchor portion can have a width of 100%, 95%, 90%, 85%, 80%, 75%, 70%, 60%, 50%, 40%, 30%, 20%, 10%, or less of the distance between the inflection points of the two support members (e.g., the width of the anchor portion can be between 100% and 50%, 100% and 70%, 100% and 80%, 90% and 70%, and 90% and 80% of the distance between inflection points of the support members).

In other aspects, the anchor portion (e.g., a closed loop) can be directly connected to a single support member.

In yet other aspects, the device can further include a shaft connected to the first end of the lateral structure, wherein the shaft is suitable to pass through a catheter for introduction into a human vein or artery. The shaft and lateral structure can be connected by a hook, latch, screw, clamp, friction fitting, adhesive, or entwined wire. For example, in certain embodiments, the first end includes a screw configured to mate with a corresponding shaft (or vice versa). The shaft can have a rectilinear (e.g., square), rhomboid, or curved (e.g., oval or circular) cross section. In certain embodiments, the shaft at one end connects to a wire, where the wire forms a loop distal to the shaft and a stem proximal to the shaft, wherein the loop connects with the first end of the lateral structure. In other embodiments, the shaft at one end terminates in a screw, wherein the screw connects with the first end of the lateral structure.

In another aspect, methods of filtering embolic material in an aorta by inserting into an aortic arch any of the above-described devices are described; positioning the filter approximately midway between an upper wall of the aortic arch and a lower wall of the aortic arch so that: the filter extends over a length approximating a distance between an innominate artery and a left subclavian artery; the lower member extends from the lateral device; and the lower member exerts lift on a middle portion of the lateral structure. The proximate portion of the upper member of the device can extend towards the ascending aorta, and an anchor portion of the upper member can extend towards the descending aorta; and anchor portion of the upper member can be positioned at a location approximating the innominate artery. Any of the foregoing methods can include lift being limited by downward force from the upper member, to preserve a horizontal position of the device within the aorta. Also, any of the foregoing methods can include the lateral structure bending downward on each of a first end and second end of the structure, and an outward force being exerted from each of such first end and the second end upon an inner wall of an ascending aorta and a descending aorta.

Any of the foregoing methods can also include embolic material being filtered from entering branch arteries of the aorta.

### BRIEF DESCRIPTION OF THE DRAWINGS

Fig. 1A is a schematic diagram of a side view of an intra-vascular device in accordance with an embodiment of the invention.
Fig. 1B is a schematic diagram of a three-quarters view of an intra-vascular device, in accordance with an embodiment of the invention.
Fig. 2 is a schematic diagram of a device installed in an aorta, in accordance with an embodiment of the invention.
Fig. 3 is a diagram of a hooked end of a device with a contact point between a loop of wire and a rest of the hook, in accordance with an embodiment of the invention.
Fig. 4 is a flow diagram of a method in accordance with an embodiment of the invention.
Fig. 5A is a diagram of a three-quarters view of an intra-vascular device with a radiopacity bead, in accordance with an embodiment of the invention.
Fig. 5B is a photograph of a radiopacity bead and clamp element for use in an embodiment of the invention.
Fig. 5C is a photograph of a cross section of Drawn Filled Tubing (DFT wire).
Fig. 5D is a schematic diagram of a side view of an intra-vascular device, in accordance with an embodiment of the invention.
Fig. 5E is a schematic diagram of a filter mesh containing DFT wire.
Fig. 6A is a photograph and a series of schematic diagrams showing various views of an intra-vascular device, in accordance with an embodiment of the invention.
Fig. 6B is a photograph and a series of schematic diagrams showing various views of an intra-vascular device, in accordance with an embodiment of the invention.
Fig. 6C is a schematic diagram showing a front view of an intra-vascular device, in accordance with an embodiment of the invention.
Figs. 7A and 7B are schematic diagrams showing side views of intra-vascular devices, in accordance with embodiments of the invention. Fig. 7A illustrates a skeleton with an increased thickness or multiple wires in order to increase the skeleton stiffness when compared with the device of Fig. 7B.
Fig. 8A is a schematic diagram showing filter meshes of the indicated pore sizes.
Fig. 8B is a schematic diagram showing perforated films with the indicated patterns, sizes, and densities of pores.
Fig. 8C is a schematic diagram showing a filter mesh with a combination of DFT and Nitinol wires.
Figs. 9A-9C are photographs showing a variety of mechanisms for connecting the intra-vascular device to a catheter.
Fig. 10 is a schematic diagram of a side view of a plunger for use in introducing intra-vascular devices of the invention into a subject, e.g., through a catheter.

### DETAILED DESCRIPTION OF THE PREFERRED EMBODIMENTS

In the following description, various embodiments of the invention will be described. For purposes of explanation, specific examples are set forth in order to provide a thorough understanding of at least one embodiment of the invention. However, it will also be apparent to one skilled in the art that other embodiments of the invention are not limited to the examples described herein. Furthermore, well-known features or processes may be omitted or simplified in order not to obscure embodiments of the invention described herein.

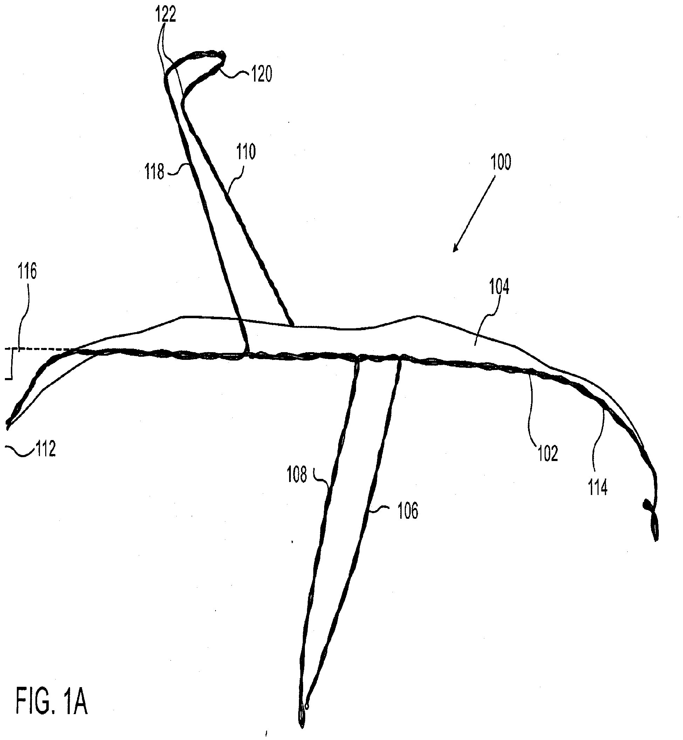

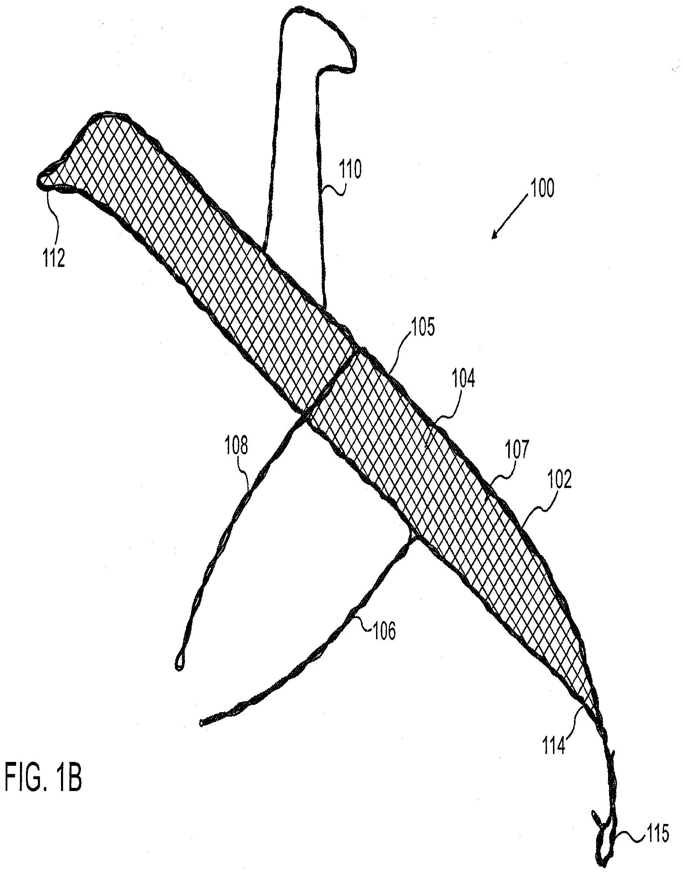

Reference is made to Figure 1A, a schematic diagram of a side-view of an intra-vascular device, and to Figure 1B, a three quarters side view of an intra-vascular device, in accordance with an embodiment of the invention. According to the invention, intravascular device 100 includes a lateral structure such as a frame or skeleton 102, a filter 104, and a series of support members such as lower members 106 and 108, and upper member 110. A first end 112 of device 100, facing upstream of blood flow in an aorta, and a second end 114 of device 100, facing downstream of blood flow in an aorta, may curve downward below a lateral plane of device 100. Second end 114 of device 100 may include a hook 115 or other clasping and retrieval section by which device 100 may be held on insertion or installation, and then snared or grasped for retrieval of device 100. In certain embodiments, the second end 114 of device 100 may include a clasp that remains connected throughout introduction and removal from a subject's aorta.

Imaginary line 116 represents a theoretical lateral plane of device 100. In some embodiments, a lateral plane of device 100 may include an approximately horizontal line tracing a middle section of skeleton 102 along device 100 before the curves of end 112 and end 114.

A first support portion 118 of upper member 110, as may be proximate to skeleton 102, may rise away from skeleton 102 at an angle towards first end 112. A second anchor portion 120 of upper member 110 doubles back on such first support portion at bend 122 and may rise upward and towards a direction of second end 114. Second anchor portion 120 of upper member 110 may taper in width towards its tip, which may be rounded or flattened.

In some embodiments, a weave of strands 107 of filter 104 may be angled at approximately 45 degrees to an outside of skeleton 102 to accommodate shifts in length or with of the structure of skeleton 102 when device 100 is installed, removed or positioned into place.

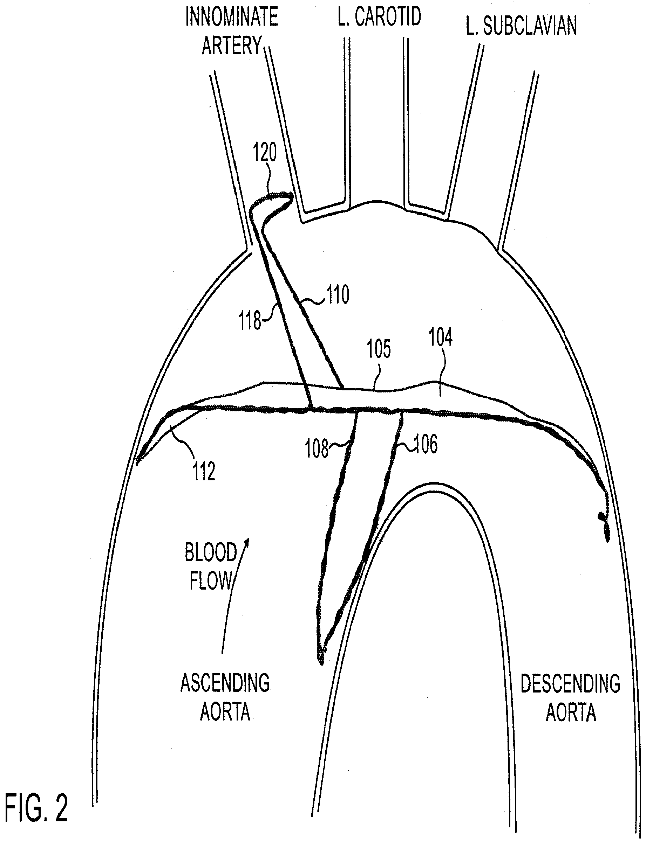

Reference is made to Figure 2, a schematic diagram of a device installed in an aorta, in accordance with an embodiment of the invention. In operation, device 100 may be installed in an aorta such that first end 112 points towards the ascending aorta. Lower members 108 and 106 may press against an internal lower wall of the ascending aorta, and such pressing may exert an upward lift force on a middle portion 105 of skeleton 102. Such lift may raise middle portion 105, and, for example, a majority, or some other portion of skeleton 102, above lateral plane 116 of skeleton 102, or may stop such middle portion 105 from sinking below lateral plane 116 of device 100. In some embodiments, lift from lower members 106 and 108 may be exerted only at such time as some other force, such as a blood flow or pulse movement, acts to push device 100 downward below lateral plane 116.

Upon deployment, installation or release, upper member 110 may extend into an innominate artery. For example, first support portion 118 of upper member 110 may come into contact with a right internal wall of the innominate artery, bend 122 may come into contact with a right internal wall of the innominate artery, and second anchor portion 120 of upper member 110 may come into contact with a superior portion of a left internal wall of the innominate artery. The multiple, possible contact or holding points of upper member 110 with the innominate artery may hold device 100 in place against a blood flow in the aorta, may prevent a roll of device 100 within the aorta, and may prevent device 100 from rising beyond a desired distance from an entry point of the innominate, left carotid and left subclavian arteries. Upper member 110 may also prevent device 100 from sliding out of position in a direction of a blood flow or of reverse flow in the aorta. Upper member 110 may exert a downward force on device 100 to counter a lift that may be exerted by lower members 106 and 108, and to keep device 100 away from the entry points of the branch arteries of the aorta, for example, as are listed above.

In some embodiments, upper member 110 may be inserted into, for example, a left subclavian artery where a curve of bend 122 may be held against a left inner wall of left subclavian artery, and second anchor portion 120 engages a counter wall.

The downward curve of first end 112 and second end 114 may likewise press against an ascending aorta and descending aorta respectively, to prevent a rise of device 100 past a desired position that approximates a midway between a lower wall of the aortic arch and an upper wall of the aortic arch. The downward curve of first end 112 and second end 114 may allow pressure to be exerted against walls of the aorta without damaging or puncturing such walls. Lower members 106 and 108 may exert a continuous lift force on skeleton 102 to keep first end 112 and second end 114 in pressure contact with an upper wall of the ascending aorta and descending aorta respectively.

In an installed position, mesh or filter 104 may block or deflect emboli or other particles from entering, for example, the three branch arteries listed above, while still preserving a space above the filter for blood to swirl and collect at such entries. The space under filter 104 may allow unfiltered blood to pass by the branch arteries of the aorta. Such space in the aorta that is left below the filter means that not all blood passing through the aorta is subject to the filtering or deflecting process of filter 104. Installation in a middle (such as between an upper wall of the aortic arch and a lower wall of the aortic arch) of the aorta rather than directly abutting an entry point into the branch arteries may allow a continued flow of blood both through the aorta and into the branch arteries, even if a portion of filter 104 is clogged with embolic or other material.

In some embodiments, lower member 106 may be connected to skeleton 102 on a first side (such as a dorsal side), and lower member 108 may be connected to skeleton 102 on a second side (such as a ventral side). A first portion of each of lower member 106 and lower member 108 that are proximate to skeleton 102 may extend in substantially parallel lines from skeleton 102. A second or lower portion of each of lower members 106 and 108, as are distal to skeleton 102 may curve towards each other at a point approximating a mid-line of skeleton 102. The lower ends of lower members 106 and 108 may terminate in, for example, small loops of the single wound strand that each of the members includes. Such curved endings may prevent a scratching or abrasion of an end of the lower member 106 or 108 against arterial tissue. The ends of each of lower members 106 and 108 may in some embodiments touch gently together though they may separate with light pressure.

In some embodiments device 100 may remain positioned in an aorta while a procedure (e.g., transcatheter aortic valve implantation) is undertaken in, for example, a heart, blood vessel, or other in-vivo area, where such procedure entails tracing a lead such as a catheter through the aorta. The ease of separation of lower members 106 and 108 may allow a removal of an arterial catheter or other device from the aorta while device 100 remains in place, and serves to deflect or filter embolic material away from entering branch arteries of the aorta.

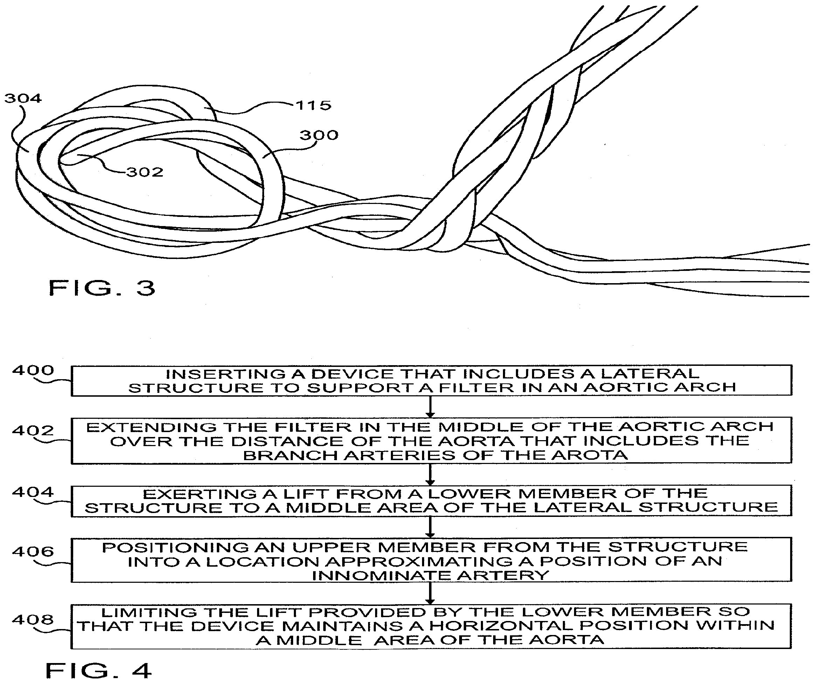

Reference is made to Figure 3, a diagram of a hooked end of a device with a contact point between a loop of wire and a rest of the hook, in accordance with an embodiment of the invention. In some embodiments, hook 115 may include a latch 300 or wire strand that may be part of a wire strand that makes up skeleton 102, and that is in contact with a rest of hook 115. In some embodiments, a wire or catheter that may end in, for example, a loop, may be threaded through latch 300 so that the loop passes between a contact point of bend 302 and curve 304. When so threaded, a wire or catheter fitted with a looped end may be clicked into hook 115, and may securely push device 100 into place or pull device out of position from an aorta. In some embodiments, the hook may end in a ball-tip so that strands from the frame do not fray or scratch the vessel wall or the inner tube of a catheter.

In some embodiments, device 100 may prevent the passage of, block, divert, or filter-out particles, such as, for example, blood clots, calcified debris or other objects that may block a flow of blood. Skeleton 102 and device 100 may also be used to support or keep in place other apparatuses.

Device 100 may be inserted into a vessel by way of, for example, a catheter, and may be threaded into, for example, a blood vessel into which device 100 may be implanted. Other methods of implanting device 100 into a blood vessel are possible. In some embodiments, device 100 may assume a shape of an extended oval or a willow leaf. Other shapes may be used.

In some embodiments, skeleton 102 may include or be constructed of, for example, Nitinol or other superelastic or shape memory alloy or material. Other materials may be used. In some embodiments, filter 104 may be or include a fine wire netting or mesh, or perforated film, such as a mesh having holes or pores of 300 microns more or less such that, for example, particles that are larger than the pores or holes are prevented from passing through the filter. Other sizes of holes or eyes may be used. In some embodiments, a shape of filter 104 may be defined or supported by a shape of skeleton 102.

In some embodiments, one or more of skeleton 102, upper member 110 and lower members 106 and 108 may be fashioned of continuous wire that has different thicknesses or properties in various areas of its lengths. For example, upper member 110 may be fashioned of a wire or portion of wire that is thin or otherwise highly flexible relative to the thickness or flexibility of one or more of lower members 106 and 108 or of other portions of skeleton 102. Such heightened flexibility may enable upper member 110 and particularly bend 122 and second portion 120 to expand or shrink upon the application of even a small force, such as, for example, the small force exerted by the contact of upper member 110 with an upper portion of a blood vessel against which it comes into contact. In contrast, lower members 106 and 108 may be fashioned of a thicker or relatively more rigid wire or filament to provide lift for a mid portion of device 100.

In some embodiments, one or more of the wires that make up upper member 110 and lower members 106 and 108 may be wound or braided around skeleton 102, and no soldered or glued connections between the wound strands of skeleton 102 and members 110, 106 and 108 may be needed.

Device 100 may be inserted or deployed through, for example, one of the branch arteries or directly through an artery in the area of the heart rather than by way of a catheter from a remote vessel.

Reference is made to Figure 4, a flow diagram of a method In block 400, there may be inserted into an aortic arch, a device that includes a lateral structure to support a filter (for example, the device of Figures 1-3, 5A, 6A-6C, 7A, or 7B may be used, or other devices described herein may be used). The length of the device may be from approximately 80 mm to 90 mm, or otherwise as may be necessary to approximate a distance between an upper wall of an ascending aorta, upstream of an opening of an innominate artery, and at an upper wall of a descending aorta downstream of an opening of a left subclavian artery. The width of the device may be from 20 mm to 35 mm, or otherwise as may approximate an internal diameter of an aorta. The device may be inserted into the aorta or introduced into a blood vessel in a collapsed form, and may assume an extended form upon its release from a tube or other insertion or positioning mechanism. In block 402, the device may extend the filter attached to the lateral structure so that the filter assumes a position approximately midway between an upper wall of the aortic arch and a lower wall of the aortic arch, and extends over the distance between the branch arteries of the aorta as are listed in block 400 above.

In block 404, a lower member connected with the device may extend downward from the lateral structure in a direction of an upstream blood-flow, and such lower member may exert a lift on a middle area of the lateral structure.

In block 406, an upper member that may be connected to the structure, may be angled in a proximate section towards upstream flow of blood in the aorta, and in a distal section relative to the device, may be angled towards downstream flow of blood in the aorta, where a bend in such upper member between the support portion and the anchor portion, may extend into a location approximating a position of an innominate artery.

In block 408, the upper member may limit the lift provided by the lower member so that the device maintains a relatively horizontal position within a middle area of the aorta.

In some embodiments, the structure is bent downward from its lateral plane on each of a first end and second end of the structure, and outward force is exerted from each of such first end and a second end of the structure upon an inner wall of an ascending aorta and descending aorta, respectively.

In some embodiments, the embolic material is filtered from entering the branch arteries of the aorta.

In some embodiments, a method may include snaring a hook at a downstream end of the device with a loop brought into contact with the hook.

An exemplary method may include separating a contact of a first side of the lower member from a second side of the lower member by pulling another device through the aorta to an area bounded by the lateral structure and the first and second sides of the lower member.

A method may include bending an inferior or lower portion of the first side of the lower member towards an inferior or lower portion of the second side of the lower member.

A method of manufacturing a device of the invention may comprise tapering or narrowing the distal end of the upper member after the bend of the upper member so that the upper member ends in a narrow, curved point.

A method may include winding a strand of structural material around the lateral structure, and extending such wound strand into one or more of the upper and lower members respectively. In some embodiments, the entire frame may be fabricated from a foil sheet that includes both the frame and the filter.

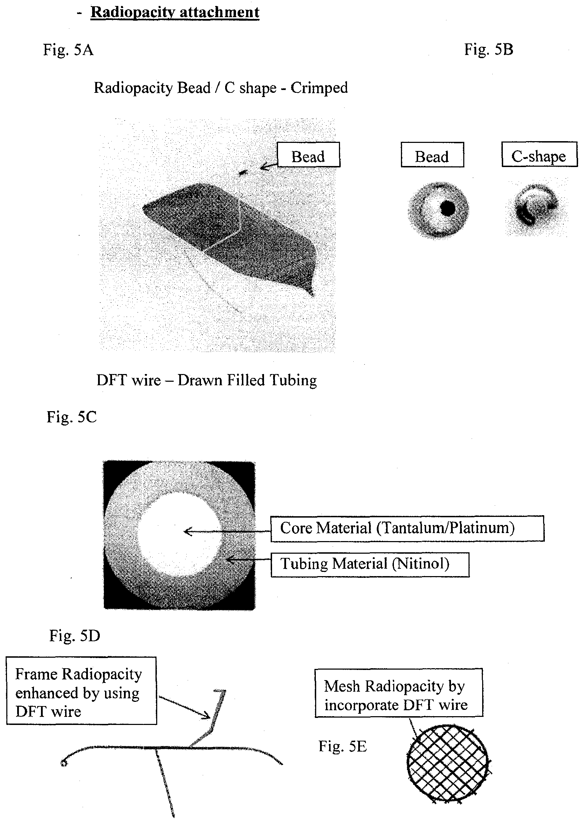

Reference is made to Figure 5. In some embodiments, it is desirable to incorporate radiopaque elements into the intra-vascular device. Such radiopaque elements can affix to, or incorporate into the skeleton of the intra-vascular device (e.g., affixed to the upper member (Figure 5A), a lower member, filter skeleton, or filter material, e.g., mesh material). The radiopaque element can be a bead or clamp (e.g., as depicted in Figure 5B). In the case of a clamp, the element can be crimped onto the intra-vascular device. In any of the embodiments of the invention, radiopaque material can be incorporated into wire forming the skeleton or filter mesh of the intra-vascular device. For example, portions of the skeleton or filter mesh can be constructed out of Drawn Filled Tubing (DFT wire). Such wire can contain, e.g., a core of tantalum and/or platinum and an outer material of, e.g., Nitinol (see, e.g., Figure 5C). In certain embodiments, the DFT wire can be incorporated into all or a portion of the intra-vascular device skeleton, upper member (e.g., as depicted in Figure 5D), either or both of the lower members, clasp, or filter mesh (e.g., as depicted in Figure 5E). In embodiments where radiopaque wire (e.g., DFT wire) is used in the filter mesh, it can be used throughout the mesh, or in a certain subset of mesh wires (e.g., as depicted in Figure 5E).

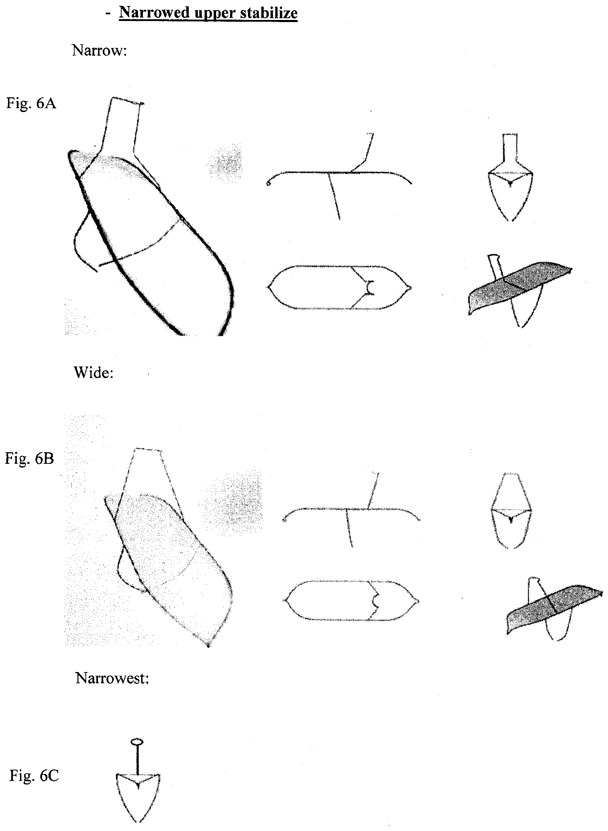

Reference is made to Figures 6A-6C. A variety of upper member structures can be used in the intra-vascular devices of the invention. Because the aortic anatomy can vary between individuals, embodiments of the intra-vascular device of the invention are shaped to adapt to a variety of aortic anatomies. Figures 6A-6C depict three different configurations for an upper member to be used in the intra-vascular devices of the invention. Each configuration contains a support portion and an anchor portion. Generally, the anchor portion will have a smaller width than the widest region of the lateral structure of the intra-vascular device. The anchor portion can have a width, e.g., of 100%, 95%, 90%, 85%, 80%, 75%, 70%, 60%, 50%, 40%, 30%, 20%, 10%, or less of the width of the widest region of the lateral structure of the intra-vascular device (e.g., the width can be between 100% and 10%, 80% and 20%, 60% and 20%, 50% and 20%, and 50% and 30% of the widest region of the lateral structure of the intra-vascular device). The anchor portion can be connected (e.g., directly connected) to a support portion of the upper member. In some embodiments, the anchor portion can be connected to a single support member of the support portion (e.g., as depicted in Figure 6C) or can be connected to multiple support members (e.g., as depicted in Figures 6A and 6B). The anchor portion can, e.g., connect two support members (e.g., as depicted in Figures 6A and 6B) or can be configured as a loop connected to a single support member (e.g., as depicted in Figure 6C). In some embodiments, the support members can be sloped towards the anchor portion. These support members can have a uniform (e.g., as depicted in Figure 6B) or non-uniform (e.g., as depicted in Figure 6A) slope. In embodiments with a non-uniform slope, the support members will contain an inflection point. At the inflection point, the distance between the support members can have, e.g., a width of 100%, 95%, 90%, 85%, 80%, 75%, 70%, 60%, 50%, 40%, 30%, 20%, 10%, or less of the width of the widest region of the lateral structure of the intra-vascular device (e.g., the width can be between 100% and 10%, 80% and 20%, 60% and 20%, 50% and 20%, and 50% and 30% of the widest region of the lateral structure of the intra-vascular device). Furthermore, the anchor portion can have a width of 100%, 95%, 90%, 85%, 80%, 75%, 70%, 60%, 50%, 40%, 30%, 20%, 10%, or less of the distance between the inflection points of the two support members (e.g., the width of the anchor portion can be between 100% and 50%, 100% and 70%, 100% and 80%, 90% and 70%, and 90% and 80% of the distance between inflection points of the support members). In certain embodiments, the width of the anchor portion and the distance between the inflection points of the two supporting members can be approximately the same. In such embodiments, the portion of the support members distal to the lateral support side of the inflection point will have substantially parallel slopes. In certain embodiments, the distal and proximal segments of each support member form a medial angle of more than 180° (e.g., as depicted in Figure 6A).

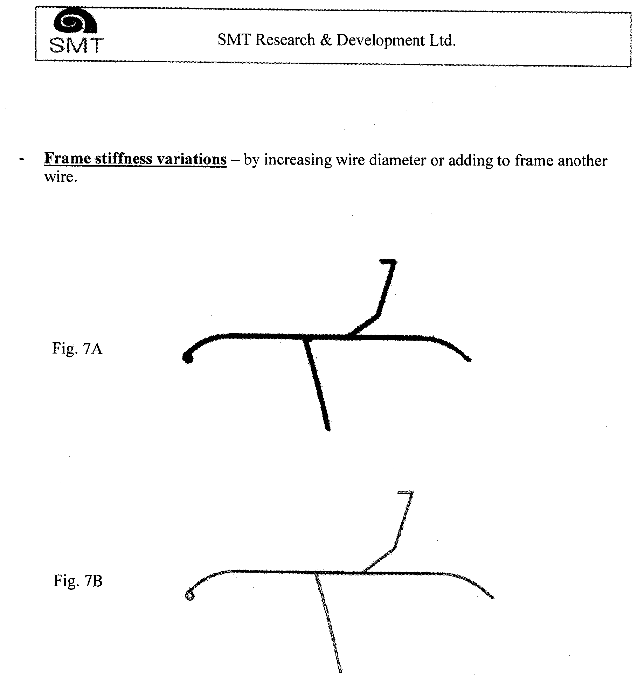

Reference is made to Figures 7A and 7B. In certain embodiments, the stiffness of the intra-vascular device will be determined by the thickness of the skeleton. For example, the skeleton can be stiffened by the inclusion of heavier gauge wire. Furthermore, multiple wires of a certain gauge can be wound together to increase the stiffness of the skeleton (e.g., the skeleton can include 2, 3, 4, 5, or more wires of to increase the stiffness of the intra-vascular device).

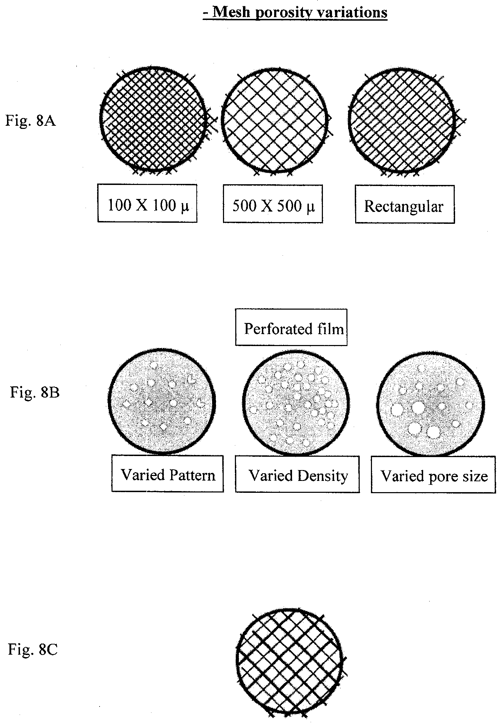

Reference is made to Figures 8A-8C. In certain embodiments of the intra-vascular device of the invention, the filter material can be a mesh (e.g., as depicted in Figures 8A and 8B) or a perforated film (e.g., as depicted in Figure 8B). In embodiments where a wire mesh is used, the wire mesh can contain square, rectangular, or rhomboid shaped pores. Each dimension of the mesh pores can be, e.g., between 50 and 1000 microns (e.g., 100, 200, 300, 400, 500, 600, or more microns; Figure 8A). In embodiments where a perforated film is present, the pores can have constant or varied pore patterns, constant or varied pore densities, and/or constant or varied pore sizes (Figure 8B).

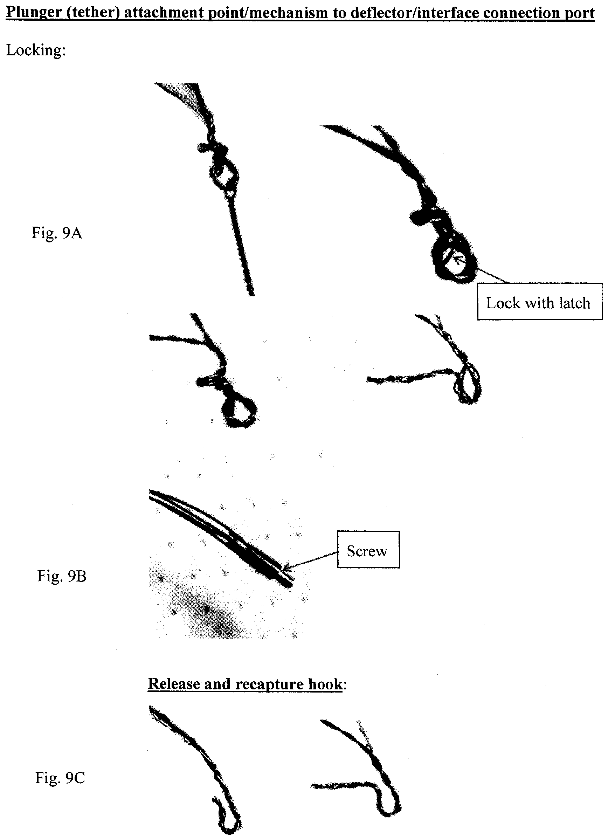

Reference is made to Figures 9A-9C. As described above, a variety of configurations can be used to connect the intra-vascular filter to a plunger (e.g., a plunger disposed within a catheter). Figure 9A depicts a locking mechanism with a latch. Figure 9B depicts a screw whereby the intra-vascular device can be mated with a screw on a plunger. Figure 9C depicts a release and recapture hook for connecting the intra-vascular device with a plunger.

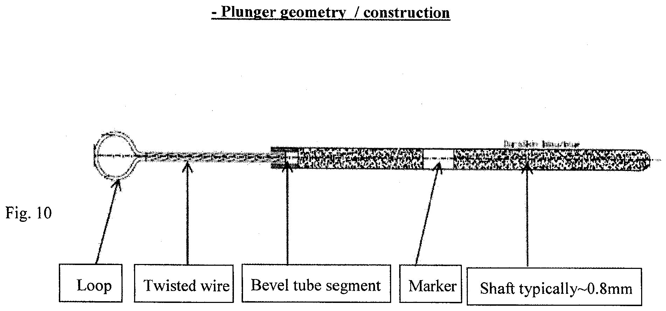

Reference is made to Figure 10. The shaft or plunger for use in connection with the device can, e.g., terminate in a loop (as depicted in Figure 10) or, e.g., a screw. In embodiments where a loop is present, the loop can be generated by winding two wires together leaving a loop at the distal end (Figure 10). The shaft or plunger can, e.g., include a radiopaque element. Furthermore, the shaft or plunger can feature a rectilinear (e.g., square) or curved (e.g., oval or circular) cross section. Differences in cross sectional shape can have advantageous properties with respect to controlling the positioning of the intra-vascular device within the aorta.

It will be appreciated by persons skilled in the art that embodiments of the invention are not limited by what has been particularly shown and described hereinabove. Rather the scope of at least one embodiment of the invention is defined by the claims below.

## Claims

1. A device to be inserted into the aortic arch for deflecting emboli, the device comprising:
a lateral structure (102) defining a lateral plane (116) comprising a first end (112) and a second end (114) to support an emboli filter (104), the lateral structure (102) has a length and the emboli filter (104) is attached to and extending the length of said lateral structure (102);
a lower member (106, 108) extending downward from said lateral structure (102) in a direction of an ascending aorta when inserted into the arch, wherein upon installation of said device, said lower member exerts lift on a middle area of said lateral structure; and
an upper member (110) extending upwards from said lateral structure (102) extending into an innominate artery upon deployment, wherein a support portion (118) of said upper member (110) being proximate to said lateral structure (102) rises away from said lateral structure (102) at an angle towards the first end (112) of said device, **characterized in** an anchor portion (120) of said upper member (110) being distal to said lateral structure (102) double back on said support portion (118) at a bend (122) and rises upward and towards a direction of the second end (114) of said device, wherein upon said installation, said upper member (110) limits said lift.

2. The device as in claim 1, wherein each of said ends ending below said lateral plane (116) of said lateral structure (102), preferably wherein said proximal end (114) includes a hook (115) configured from a wire of said lateral structure (102), said hook (115) having a latch (300) to hold a lasso brought into contact with said hook (115); or
said lower member (106, 108) comprises a right segment (106) having a first end attached to said lateral structure (102), and a left segment (108) having a first end attached to said lateral structure (102), and wherein a second end of said right segment (106) is unconnected to a second end of said left segment (108), preferably wherein said second end of said right segment (106) is angled towards said second end of said left segment (108).

3. The device as in claim 1, wherein said anchor portion (120) of said upper member (110) tapers distal to said lateral structure (102), or wherein said upper member (110) and said lower member (106, 108) are extensions of a wire entwined around said lateral structure (102).

4. The device as in any of the preceding claims, wherein said device further comprises a radiopacity marker, preferably wherein said radiopacity marker is a bead or a clamp.

5. The device as in any of the preceding claims, wherein said support portion (118) comprises two support members that slope from said lateral structure (102) to said anchor portion (120).

6. The device as in claim 5, wherein said slope of each of said two members is non-uniform and comprises an inflection point thereby forming a segment of each of said two members proximal to said lateral structure (102) and a segment of said two members distal to said lateral structure (102), preferably wherein at said inflection point, the segments of each of said two members of said support portion (118) form a medial angle of morethan 180°.

7. The device as in claim 6, wherein the distance between said inflection points of said two members is less than the width of the widest region of said lateral structure (102), preferably wherein the distance between said inflection points is less than 50% of the width of the widest region of said lateral structure (102), or the distance between said inflection points is less than 30% of the width of the widest region of said lateral structure (102).

8. The device as claim 7, wherein the width of said anchor portion (120) is the same as the distance between said inflection points.

9. The device as in claim 7, wherein the width of said anchor portion (120) is less than the distance between said inflection points, preferably wherein
the width of said anchor portion (120) is between 80% and 100% of the distance between said inflection points, or
the width of said anchor portion (120) is less than 80% of the distance between said inflection points.

10. The device as in any of the preceding claims, wherein said anchor portion (120) is directly connected to a single support member (118), preferably wherein said anchor portion (120) is a closed loop.

11. The device as in any of the preceding claims, wherein said filter (104) is a mesh.

12. The device as in claim 11, wherein the pores in said mesh are rectilinear, preferably wherein the pores in said mesh are square.

13. The device as in any of claims 2 to 12, wherein said first end (114) is configured to mate with a corresponding shaft.

14. The device as in any of claims 2 to 13, further comprising a shaft connected to said first end (114) of the lateral structure (102), wherein said shaft is suitable to pass through a catheter for introduction into a human vein or artery, preferably wherein said shaft is connected to said first end (114) of the lateral structure (102) by a hook (115), latch, screw, clamp, friction fitting, adhesive, or entwined wire.

15. The device as in claim 14, wherein said shaft has a rectilinear cross section, preferably wherein said shaft has a square cross section.

16. The device as in claim 14, wherein said shaft has an oval or circular cross section.

17. The device as in any of claims 14 to 16, wherein said shaft at one end connects to a wire, wherein said wire forms a loop distal to the shaft and a stem proximal to said shaft and wherein said loop is adapted to connect with said first end (114) of the lateral structure (102), or wherein said shaft at one end terminates in a screw that connects with said first end (114) of the lateral structure (102).

## Patentansprüche

1. Eine in den Aortenbogen einzuführende Vorrichtung zur Ablenkung von Embolien, bestehend aus:
einer seitlichen Struktur (102), die eine seitliche Ebene (116) beschreibt und ein erstes Ende (112) und ein zweites Ende (114) als Halterung eines Filters für embolische Debris (104) umfasst, wobei die seitliche Struktur (102) eine Länge aufweist und der Filter für embolische Debris (104) an der Länge der besagten seitlichen Struktur (102) befestigt ist und darüber hinausragt,
einem unteren Element (106, 108), das sich bei Einführung in den Bogen von der besagten seitlichen Struktur (102) hin zu einer aufsteigenden Aorta erstreckt, wobei nach Anlegen der besagten Vorrichtung das besagte untere Element eine Anhebung des mittleren Bereichs der besagten seitlichen Struktur bewirkt, und
einem oberen Element (110), das sich beim Einsatz von der besagten seitlichen Struktur (102) nach oben in eine unbekannte Arterie erstreckt, wobei ein Halteabschnitt (118) des besagten oberen Elements (110) in der Nähe der besagten seitlichen Struktur (102) liegt und winklig von der besagten seitlichen Struktur (102) in Richtung des ersten Endes (112) der besagten Vorrichtung hervorsteht, **dadurch gekennzeichnet, dass**
ein Ankerabschnitt (120) des besagten oberen Elements (110) distal zur besagten seitlichen Struktur (102) liegt und auf dem besagten Halteabschnitt (118) einen Bogen (122) beschreibt und sich nach oben in Richtung auf das zweite Ende (114) der besagten Vorrichtung erstreckt, wobei das besagte obere Element (110) bei der besagten Anbringung die besagte Anhebung begrenzt.

2. Die Vorrichtung gemäß Anspruch 1, wobei jedes der besagten Enden unter der besagten seitlichen Ebene (116) der besagten seitlichen Struktur (102) endet und vorzugsweise das besagte proximale Ende (114) einen Haken (115) umfasst, der aus einem Draht der besagten seitlichen Struktur (102) besteht und der besagte Haken (115) eine Klinke (300) aufweist, um ein mit dem besagten Haken (115) in Kontakt gebrachtes Lasso zu halten, oder
das besagte untere Element (106, 108) ein rechtes Segment (106) mit einem ersten, an der besagten seitlichen Struktur (102) befestigten Ende umfasst, sowie ein linkes Segment (108) mit einem ersten, an der besagten seitlichen Struktur (102) befestigten Ende und wobei das zweite Ende des besagten rechten Segments (106) nicht mit einem zweiten Ende des besagten linken Segments (108) verbunden ist, wobei vorzugsweise das besagte zweite Ende des besagten rechten Elements (106) in Richtung auf das besagte zweite Ende des besagten linken Segments (108) abgewinkelt ist.

3. Die Vorrichtung gemäß Anspruch 1, wobei der besagte Ankerabschnitt (120) des besagten oberen Elements (110) sich distal zur besagten seitlichen Struktur (102) hin verjüngt oder wobei das besagte obere Element (110) und das besagte untere Element (106, 108) Verlängerungen eines um die besagte seitliche Struktur (102) gewickelten Drahtes sind.

4. Die Vorrichtung gemäß einem der vorstehenden Ansprüche, wobei die besagte Vorrichtung weiterhin einen röntgenopaken Marker aufweist und der röntgenopake Marker vorzugsweise eine Kugel oder eine Klammer ist.

5. Die Vorrichtung gemäß einem der vorstehenden Ansprüche, wobei der besagte Halteabschnitt (118) zwei Halteelemente aufweist, die sich von der besagten lateralen Struktur (102) zum besagten Ankerabschnitt (120) hin neigen.

6. Die Vorrichtung gemäß Anspruch 5, wobei die besagte Neigung jedes der besagten beiden Elemente nicht gleichmäßig ist und einen Wendepunkt aufweist, wodurch ein Segment jedes der beiden besagten Elemente proximal zur besagten seitlichen Struktur (102) und ein Segment jedes der beiden besagten Elemente distal zur besagten seitlichen Struktur (102) gebildet werden, wobei die Segmente jedes der beiden besagten Elemente des besagten Halteabschnitts (118) vorzugsweise am besagten Wendepunkt einen medialen Winkel von über 180° bilden.

7. Die Vorrichtung gemäß Anspruch 6, wobei die Distanz zwischen den besagten Wendepunkten der besagten zwei Elemente kleiner als die Breite an der breitesten Stelle der besagten seitlichen Struktur (102) ist, wobei vorzugsweise die Distanz zwischen den besagten Wendepunkten unter 50 % der Breite an der breitesten Stelle der besagten seitlichen Struktur (102) liegt oder die Distanz zwischen den besagten Wendepunkten unter 30 % der Breite an der breitesten Stelle der besagten seitlichen Struktur (102) liegt.

8. Die Vorrichtung gemäß Anspruch 7, wobei die Breite des besagten Ankerabschnitts (120) der Distanz zwischen den besagten Wendepunkten entspricht.

9. Die Vorrichtung gemäß Anspruch 7, wobei die Breite des besagten Ankerabschnitts (120) unter der Distanz zwischen den besagten Wendepunkten liegt, wobei vorzugsweise die Breite des besagten Ankerabschnitts (120) 80 bis 100 % der Distanz zwischen den besagten Wendepunkten entspricht oder die Breite des besagten Ankerabschnitts (120) unter 80 % der Distanz zwischen den besagten Wendepunkten liegt.

10. Die Vorrichtung gemäß einem der vorstehenden Ansprüche, wobei der besagte Ankerabschnitt (120) direkt mit einem einzelnen Halteelement (118) verbunden ist, wobei es sich bei dem besagten Ankerabschnitt (120) vorzugsweise um eine geschlossene Schleife handelt.

11. Die Vorrichtung gemäß einem der vorstehenden Ansprüche, wobei es sich bei dem besagten Filter (104) um ein Netz handelt.

12. Die Vorrichtung gemäß Anspruch 11, wobei die Poren in dem besagten Netz rechtlinig verlaufen, vorzugsweise quadratisch.

13. Die Vorrichtung gemäß einem der Ansprüche 2 bis 12, wobei das besagte erste Ende (114) so ausgelegt ist, dass es sich mit einer entsprechenden Achse zusammenfügt.

14. Die Vorrichtung gemäß einem der Ansprüche 2 bis 13, die weiterhin eine mit dem besagten ersten Ende (114) der seitlichen Struktur (102) verbundene Achse aufweist, wobei die besagte Achse geeignet ist, durch einen Katheter zu passen, um in eine menschliche Vene oder Arterie eingeführt zu werden, wobei die besagte Achse über einen Haken (115), eine Klinke, eine Schraube, eine Klammer, eine Reibpassung, Klebeband oder Wickeldraht mit dem besagten ersten Ende (114) der seitlichen Struktur (102) verbunden ist.

15. Die Vorrichtung gemäß Anspruch 14, wobei die besagte Achse einen rechteckigen Querschnitt aufweist, vorzugsweise einen quadratischen Querschnitt.

16. Die Vorrichtung gemäß Anspruch 14, wobei die besagte Achse einen ovalen oder kreisförmigen Querschnitt aufweist.

17. Die Vorrichtung gemäß einem der Ansprüche 14 bis 16, wobei die besagte Achse an einem Ende mit einem Draht verbunden ist, wobei der besagte Draht eine zur Achse distale Schlaufe bildet und einen zur besagten Achse proximalen Stiel, wobei die besagte Schlaufe geeignet ist, mit dem besagten ersten Ende (114) der seitlichen Struktur (102) verbunden zu werden oder wobei die besagte Achse an einem Ende in einer Schraube endet, die mit dem besagten ersten Ende (114) der seitlichen Struktur (102) verbunden ist.

## Revendications

1. Dispositif à insérer dans l'arc aortique afin de dévier des emboles, le dispositif comprenant :
une structure latérale (102) définissant un plan latéral (116) comprenant une première extrémité (112) et une seconde extrémité (114) pour supporter un filtre d'emboles (104), la structure latérale (102) présente une longueur et le filtre d'emboles (104) est fixé et s'étend sur la longueur de ladite structure latérale (102) ;
un élément inférieur (106, 108) s'étendant vers le bas depuis ladite structure latérale (102) selon une direction d'une aorte ascendante lorsqu'il est inséré dans l'arc, où lors de la mise en place dudit dispositif, ledit élément inférieur exerce une poussée vers le haut sur une zone centrale de ladite structure latérale ; et
un élément supérieur (110) s'étendant vers le haut depuis ladite structure latérale (102) s'étendant dans un tronc brachiocéphalique lors du déploiement, où une partie de support (118) dudit élément supérieur (110) étant à proximité de ladite structure latérale (102) remonte en s'écartant de ladite structure latérale (102) selon un angle en direction de la première extrémité (112) dudit dispositif, **caractérisé en ce qu'**une partie d'ancrage (120) dudit élément supérieur (110) étant distale par rapport à ladite structure latérale (102) fait un brusque crochet par rapport à ladite partie de support (118) au niveau d'un coude (122) et remonte vers le haut et en allant en direction de la deuxième extrémité (114) dudit dispositif, où lors de ladite mise en place, ledit élément supérieur (110) limite ladite poussée vers le haut.

2. Dispositif selon la revendication 1, dans lequel chacune desdites extrémités se terminent sous ledit plan latéral (116) de ladite structure latérale (102), de préférence dans lequel ladite extrémité proximale (114) inclut un crochet (115) configuré à partir d'un fil de ladite structure latérale (102), ledit crochet (115) ayant un élément de verrouillage (300) pour maintenir un lasso amené en contact avec ledit crochet (115) ; ou
ledit élément inférieur (106, 108) comprend un segment droit (106) présentant une première extrémité fixée sur ladite structure latérale (102), et un segment gauche (108) présentant une première extrémité fixée sur ladite structure latérale (102), et où une seconde extrémité dudit segment droit (106) est non reliée à une seconde extrémité dudit segment gauche (108), de préférence où ladite seconde extrémité dudit segment droit (106) est inclinée vers ladite seconde extrémité dudit segment gauche (108).

3. Dispositif selon la revendication 1, dans lequel ladite partie d'ancrage (120) dudit élément supérieur (110) s'effile de manière distale par rapport à ladite structure latérale (102), ou bien dans lequel ledit élément supérieur (110) et ledit élément inférieur (106, 108) sont des extensions d'un fil entrelacé autour de ladite structure latérale (102).

4. Dispositif selon l'une quelconque des revendications précédentes, où ledit dispositif comprend en outre un marqueur radio-opaque, de préférence où ledit marqueur radio-opaque est une bille ou une pince.

5. Dispositif selon l'une quelconque des revendications précédentes, dans lequel ladite partie de support (118) comprend deux éléments de support qui s'inclinent depuis ladite structure latérale (102) en direction de ladite partie d'ancrage (120).

6. Dispositif selon la revendication 5, dans lequel ladite inclinaison de chacun desdits deux éléments est non uniforme et comprend un point d'inflexion formant ainsi un segment de chacun desdits deux éléments proximal par rapport à ladite structure latérale (102) et un segment desdits deux éléments distal par rapport à ladite structure latérale (102), de préférence où au niveau dudit point d'inflexion, les segments de chacun desdits deux éléments de ladite partie de support (118) forment un angle médial de plus de 180°.

7. Dispositif selon la revendication 6, dans lequel la distance entre lesdits points d'inflexion desdits deux éléments est inférieure à la largeur de la région la plus large de ladite structure latérale (102), de préférence dans lequel la distance entre lesdits points d'inflexion est inférieure à 50 % de la largeur de la région la plus large de ladite structure latérale (102), ou la distance entre lesdits points d'inflexion est inférieure à 30 % de la largeur de la région la plus large de ladite structure latérale (102).

8. Dispositif selon la revendication 7, dans lequel la largeur de ladite partie d'ancrage (120) est égale à la distance entre lesdits points d'inflexion.

9. Dispositif selon la revendication 7, dans lequel la largeur de ladite partie d'ancrage (120) est inférieure à la distance entre lesdits points d'inflexion, de préférence dans lequel la largeur de ladite partie d'ancrage (120) est comprise entre 80 % et 100 % de la distance entre lesdits points d'inflexion, ou la largeur de ladite partie d'ancrage (120) est inférieure à 80 % de la distance entre lesdits points d'inflexion.

10. Dispositif selon l'une quelconque des revendications précédentes, dans lequel ladite partie d'ancrage (120) est directement reliée à un unique élément de support (118), de préférence dans lequel ladite partie d'ancrage (120) est une boucle fermée.

11. Dispositif selon l'une quelconque des revendications précédentes, dans lequel ledit filtre (104) est un treillis.

12. Dispositif selon la revendication 11, dans lequel les pores dans ledit treillis sont rectilignes, de préférence dans lequel les pores dans ledit treillis sont carrés.

13. Dispositif selon l'une quelconque des revendications 2 à 12, dans lequel ladite première extrémité (114) est configurée pour s'accoupler avec un axe correspondant.

14. Dispositif selon l'une quelconque des revendications 2 à 13, comprenant en outre un axe relié à ladite première extrémité (114) de la structure latérale (102), où ledit axe est approprié pour passer à travers un cathéter pour une introduction dans une veine ou artère humaine, de préférence où ledit axe est relié à ladite première extrémité (114) de la structure latérale (102) par un crochet (115), un élément de verrouillage, une vis, une pince, un ajustement serré, un adhésif, ou un fil entrelacé.

15. Dispositif selon la revendication 14, dans lequel ledit axe présente une section transversale rectiligne, de préférence dans lequel ledit axe présente une section transversale carrée.

16. Dispositif selon la revendication 14, dans lequel ledit axe présente une section transversale ovale ou circulaire.

17. Dispositif selon l'une quelconque des revendications 14 à 16, dans lequel ledit axe à une première extrémité est relié à un fil, où ledit fil forme une boucle distale par rapport audit axe et une tige proximale par rapport audit axe et où ladite boucle est conçue pour être reliée à ladite première extrémité (114) de la structure latérale (102), ou dans lequel ledit axe à une première extrémité se termine en une vis qui est reliée à ladite première extrémité (114) de la structure latérale (102).
